# EUROPEAN PATENT SPECIFICATION

(11) **EP 0 357 646 B1**
(45) Date of publication and mention of the grant of the patent: **22.03.1995**
(21) Application number: 88903704.0
(22) Date of filing: 05.04.1988
(51) Int. Cl.: A61K 31/07, A61K 31/20

(54) **TREATMENT OF AGED SKIN WITH ORAL 13-CIS-RETINOIC ACID**
BEHANDLUNG GEALTERTER HAUT MIT ORALER 13-CIS-RETINSÄURE
TRAITEMENT PAR VOIE ORALE DE LA PEAU AGEE AVEC DE L'ACIDE 13-CIS-RETINOIQUE

(30) Priority: 06.04.1987 US 35544
(43) Date of publication of application: 14.03.1990
(73) Proprietor: DALTEX MEDICAL SCIENCES, INC., Fairfield, NJ 07006 (US)
(72) Inventor: PLEWIG, Gerd, D-4000 Düsseldorf 30 (DE); KLIGMAN, Albert, M., Philadelphia, PA 19106 (US)
(74) Representative: Koepsell, Helmut, Dipl.-Ing.
(86) International application number: US8801103
(87) International publication number: WO8807857

(56) References cited:
- GB-A- 1 335 867
- Retinoids, ED.; C.E. ORFANOS et al., published 29 July 1981, pp. 219-223, 232-235, see entire document
- DRUGS, vol. 28, 1984, pages 6-37, ADIS Press Ltd.; A. WARD et al.: "Isotretionoin. A review of its pharmacological properties and therapeutic efficacy in acne and other skin disorders"
- HAUTARTZT, vol. 35, 1984, pages 623-629, C. Springer-Verlag, DE; R. CÖRLIN et al.: "13-cis-retin-säure. Niedrig dosierte orale Anwendung bei Acne papulopustulosa"
- DERMATOLOGICA, vol. 171, 1985, pages 213-219, S. KARGER AG, Basel, CH; J. GARIQUE et al.: "Action de l'acide 13-cis-rétinoique et du rétinoide aromatique sur divers paramètres cutanés, chez le rat rendu hyperséborrhéique"
- MAYO CLINICAL PROC., vol. 57, 1982, pages 51-57, C.H. DICKEN et al.: "Systemic retinoids in dermatology"
- Z. HAUTKR., vol. 57, no. 15, 1982, pages 1137-1143, Grosse Verlag, Berlin, DE; E. HOTING et al.: "Systemische Retionoidtherapie - Wirkprinzipien und klinische Erfahrungen bei erythematosquamösen und anderen Dermatosen"
- INT. J. DERMATOL., vol. 25, no. 10, December 1986, pages 660-663; E. HOTING et al.: "Treatment of Rosacea with isotretinoin"
- J. AM. ACAD. DERMATOL., vol. 6, no. 4, 1982, pt 2, suppl., pages 766-785; G. PLEWIG et al.: "Action of isotretinoin in acne rosacea and gramnegative folliculitis"
- ACTA VITAMINOL. ENZYMOL., vol. 6, no. 4, 1984, pages 325-337; M. GANDOLA: "La terapia dell'acne grave e della Rosacea con acido 13-cis-retinoico (Isotretinoin) per via orale"

## Description

### Field of the Invention

The present invention relates to the treatment of various associated disorders of the skin which are typical of many elderly persons. More particularly, the invention is directed to the treatment of such disorders with low oral doses of 13-cis-retinoic acid.

### Background of the Invention

Many older persons (i.e., generally over 50 years of age and referred to herein as "the elderly") have "problem" skin deriving from multiple influences. These increase steadily with age, and all are worsened by years of earlier excessive exposure to sunlight. The sun is a significant contributor to chronic skin problems which are very prevalent in the aging population. These skin problems are most vividly expressed in the most exposed areas of the skin, and especially on the face, an area rich in blood vessels, follicles, and sebaceous glands. The face is also a highly permeable region with a poor barrier layer, allowing ready penetration of potentially toxic chemicals originating in soaps, cosmetics, fragrances, toiletries, etc. The face is under continuous chemical assault, as well as radiation insults. The end result is abnormal skin, a consequence of multiple factors acting in combination. One can recognize the following clinical disorders:
1) Rosacea-like syndromes with persistent redness, papules, and solid edema-like swellings;
2) Hyperplasia of the sebaceous glands;
3) A seborrheic-like dermatitis, peculiar to the elderly;
4) Pityrosporon-induced folliculitis;
5) Infestation by Demodex mites, with mite-associated folliculitis, erythema, and scaling;
6) Enlarged, roughened nose (rhinophyma ;
7) Overgrowth of P. acnes, an anaerobic bacteria living in the follicles;
8) Flat, keratotic and pigmented seborrheic keratoses;
9) Rough, scaly lesions of the actinic keratoses type;
10) Scaly, flaky, dandruff-like conditions of the scalp; and
11) Trichostasis spinulosa--follicular impactions on nose, cheeks, temples and nape of the neck.
Changes take place against a background of photoaged skin showing yellowing, wrinkling, dryness, dilated vessels and comedones.

U.S. Patent 4,603,146 of Albert M. Kligman discloses the use of vitamin A acid (retinoic acid or tretinoin) as a topical medication for sun-damaged skin of adults, usually starting about middle age, which acts to reverse and retard the effects of photoaging through its actions on the damaged connective tissue and abnormal epidermis. Further, pending U.S. Patent Application Serial No. 886,595 of Albert M. Kligman for "Methods for Treatment of Sundamaged Human Skin with Retinoids" discloses the topical use of various retinoic acid derivatives, including 13-cis-retinoic acid, for treating photoaged skin.

13-cis-retinoic acid, more commonly referred to simply as cis-retinoic acid or isotretinoin or by the trademarks "ACCUTANE" (in U.S.) or "ROACCUTAN" (in Europe), is an FDA-approved oral medication for the treatment of severe, refractory acne vulgaris, in particular acne conglobata. Cis-retinoic acid is also used, but not FDA-approved, for the treatment of certain chronic, uncurable inflammatory and scaling disorders, viz, psoriasis, ichthyosis, Darier's disease, lupus erythematosus, granulomatous rosacea, etc. Orally administered isotretinoin also has anti-tumor activity in very high doses and can bring about at least partial regression of actinic keratoses, keratoacanthomas, and basal cell carcinomas.

In such prior art treatments the recommended oral doses for acne are generally in the range of 40 to 80 mg daily (0.5-2.0 mg/kg body weight per day), and higher doses, sometimes as much as 200 mg per day, are often given for other chronic, disabling disorders. At these high dosages adverse side reactions occur in all patients and are recognized as the general signs of hypervitaminosis A, namely dry skin, conjunctivitis, cheilitis, nasal bleeding, fragile skin, hair loss, pruritus, muscle pains, hyperostosis of bone, calcification of ligaments, gastrointestinal upsets, pseudotumor cerebri and others.

In a certain proportion of patients taking oral isotretinoin, blood tests show potentially serious abnormalities, viz, increased cholesterol, reduction of high density lipoproteins (which protect against coronary heart disease), increased levels of triglycerides, elevation of hepatic enzymes, xanthoma formation and others. Moreover, cis-retinoic acid has been found to be extremely teratogenic.

Lower doses of cis-retinoic acid in the range of 0.05 or 0.1 mg/kg body weight per day, have been reported to be beneficial in acne conglobata (nodulo-cystic acne). Corlin, R. et al., "13-cis-retinoic acid. Low Dosage Oral Use in Acne Papulopustulosa. Results of a Multicenter Study," Hautarzt 35:623-629 (1984); Ward, A. et al., "Isotretinoin. A Review of its Pharmaceutical Properties and Therapeutic Efficacy in Acne and Other Skin Disorders," Drugs 28:6-37. (1984). The latter publication notes that the pharmacological profile of isotretinoin suggests that it acts primarily by reducing sebaceous gland size and sebum production, and as a result alters the composition of the surface lipids. The follicular microflora are also reduced, owing to diminished sebum production. The reference also reports encouraging preliminary results in small numbers of patients with rosacea, gram-negative folliculitis, Darier's disease, ichthyosis and pityriasis rubra pilaris. The response in keratinizing disorders resembles that of the related drug etretinate, another retinoic acid derivative.

Plewig, G. et al., "Effects of Two Retinoids in Animal Experiments and After Clinical Application in Acne Patients: 13-Cis-Retinoic Acid Ro4-3780 and Aromatic Retinoid Ro10-9359," Retinoids, Pages 219-235 (Springer-Verlag 1981) discloses clinical studies of sixty patients with severe ("untreatable") acne using oral doses of 13-cis-retinoid acid ranging from 0.05 to 2.0 mg/kg body weight. The authors reported a dramatic but dose-dependent response in all patients, where with lower doses the improvement showed delayed onset. An anti-inflammatory response was said to be demonstrated.

### Brief Summary of the Invention

According to the present invention, a number of skin disorders most often found in elderly persons (over 50 years of age) may be improved, moderated, and partially reversed by the oral administration of unusually low doses of 13-cis-retinoic acid. These doses are therapeutically effective, but lack cutaneous or systemic toxicity, and are probably non-teratogenic as well. The disorders that respond to the very low doses are often non-descript, representing mixtures of conditions that are not clearly classified in modern dermatological textbooks and are aggravated by photoaging. This new oral medication is a true treatment modality, not just of photodamaged skin, but of a group of associated conditions in the elderly.

Specific indications against which the present invention is therapeutically effective include:
1) Sebaceous gland enlargement, giving the skin a knobby, pebbly texture, sometimes taking the form of circumscribed growths, resembling localized tumors, viz. circumscribed sebaceous hyperplasia of the forehead;
2) Demodex infestation of follicles, with ensuing folliculitis, perifollicular erythema, and scaling;
3) Horny impactions of follicles leading to conspicuous pores and retention of fine hairs and horn (follicular hyperkeratosis), retention of comedo-like follicular plugs (trichostasis spinulosa), particularly on the nose, cheeks, temples and nape of the neck;
4) Leakage of sebum out of follicles whose walls have been weakened by a dense growth of yeast-like fungi Pityrosporon species and especially anaerobic P. acnes, producing papules and pustules. Unlike acne, these do not originate from ruptured comedones. This reaction is a folliculitis due to sebum leakage;
5) Flushing and redness, associated with dilated vessels, resembling rosacea, accompanied by sensitive skin which stings and burns;
6) Scaling and redness along the sides of the nose and cheeks, also the eyebrows, mimicking and related to seborrheic dermatitis of younger persons;
7) Flat, brown pigmented keratotic lesions of the seborrheic keratosis variety;
8) Rough, scaly lesions of the actinic keratosis type;
9) Scaly, flaky, dandruff-like conditions of the scalp;
10) Fine wrinkles caused by dryness or loss of moisture from the aged skin; and
11) Accelerated hair loss due to seborrheic dermatitis and perhaps other subclinical inflammatory components of patterned baldness in males and females (androgenic familial alopecia).

The unusually low doses, preferably given in capsule form, are in the range of about 1 to 5 mg daily or every other day, and even lower doses are possible for maintenance therapy. Though 1 or 2 mg daily may be below the teratogenic level, the treatment of females should be limited to post-menopausal and non-childbearing women (that is, women of childbearing potential must not be given this drug). Males over 50 years of age are eligible for treatment.

### Detailed Description of Preferred Embodiments

In the practice of the present invention, a therapeutically effective but low dose of 13-cis-retinoic acid is administered orally which substantially avoids all the adverse side effects of hypervitaminosis A induced by higher, conventional therapeutic doses of cis-retinoic acid in severe acne and chronic dermatoses. The effective doses according to the present invention are one-tenth to one-twentieth or less the doses of prior art treatments. Although conventional doses of isotretinoin are prescribed in milligrams per kilogram of body weight, the doses of the present invention are so low that it is irrelevant to take body weight into account.

It has been surprisingly found that doses of about 5 mg or less daily, and as low as 1 or 2 mg daily or every other day are effective while providing the least danger of toxicity and teratogenicity.

Isotretinoin is available commercially, being sold by Roche Laboratories under the trademark "ACCUTANE." Normally an oily suspension, it may suitably be administered in capsule form containing the required daily or less frequent dose. The oral dosage form may additionally comprise pharmaceutically acceptable diluents, flavoring agents, lubricants, and other suitable excipients which are standard in the art. For example, suitable diluents for inclusion in filled capsules include, but are not limited to lactose, mannitol, calcium carbonate and magnesium carbonate. Other forms of oral administration will be evident to those of ordinary skill in the art.

Although no signs of toxicity or teratogenicity have been found with the low doses of the present invention, the low dose treatment regimen of the present invention should be limited to older women beyond childbearing potential, non-ovulating, mainly post-menopausal, ovariectomized or after tubal ligation, to avoid any possible teratogenicity. The treatment may be used without restriction to older men. Thus, in order to maintain the therapeutic effects of the present invention, the treatment must be continued for long periods, such as six months to two years, and preferably indefinitely, i.e., a program of maintenance therapy should be used since significant residual effects are not generally obtained at such low dosages. Maintenance doses after achieving a therapeutic benefit may be 1 to 2.5 mg or even less two or three times a week.

The oral administration of isotretinoin according to the present invention may be combined with other therapies, such as low strength topical retinoic acid for even greater effects in treating and lessening the complications of photoaged skin. While retinoic acid irritates many patients when applied topically, oral administration of isotretinoin is very well tolerated. Topical tretinoin makes rosacea and seborrheic dermatitis worse. A further advantage of oral isotretinoin is that topical retinoic acid has no appreciable effect on enlarged sebaceous glands. Many of the indications for low-dose oral cis-retinoic acid are different than topical tretinoin.

More than one hundred (100) women and men have been treated according to the invention with oral cis-retinoic acid at doses of 2.5 to 5 mg daily for periods ranging from six (6) months to two (2) years. Even lower doses were successfully used in other patients. The results obtained were at a slower rate, but after four (4) to six (6) months, the results were about equivalent to similar patients treated with 40 mg cis-retinoic acid daily. The following results were obtained with these low doses for the disorders indicated, which are primarily inflammatory disorders of the elderly:

### (a) Rosacea-like dermatitis

Rosacea, or a condition similar to it, increases with age and is quite common in women and men past 50. Clinical signs and symptoms include: redness, scaliness, papulo-pustules, "sensitive" skin (stinging and burning from fragrances, soaps, astringents, etc.), enlarged vessels (telangiectasia), sometimes with enlargement of the nose (rhinophyma). Minor forms are usually missed by physicians. Patients are often flusher-blushers with unstable vascular systems. Flushing may be induced by heat, cold, wind, emotions, wine, alcoholic spirits of various kinds, hot drinks, spices, etc. Photophobia with blepharitis and conjunctivitis can frequently be found when looked for. Many patients go from doctor to doctor with "problem skin," which by history and inspection can be properly classified as rosacea, a variable syndrome.

Topical retinoic acid aggravates rosacea because of its irritant effect and cannot be used for this indication. Low-dose cis-retinoic acid moderates all of these manifestations, viz, the eye problems, the pimples, the redness and blushing, the unusual sensitivity to chemicals and sunlight. This is a very strong indication for a problem that is not properly diagnosed or recognized, even by specialists. Dilated vessels, cosmetically troublesome, become less conspicuous.

### (b) Enlarged sebaceous glands

Hyperplasia of sebaceous glands occurs in various forms including: (1) circumscribed sebaceous hyperplasia of the forehead and (2) whitish-yellowish, small nodules, often in linear array, on the neck and face, especially in photodamaged skin, (so-called stippled skin-erythrosis interfollicularis colli). Sebaceous hyperplasia coarsens facial skin and makes the surface pebbly. One of the most dramatic effects of cis-retinoic acid orally, unobtainable topically by any other retinoid, is shrinkage of the sebaceous gland. The enlarged glands in older persons do not secrete more sebum; oiliness is not the problem. It is their size that is aesthetically unpleasing and unattractive. Low-dose cis-retinoic acid according to the present invention reduces the size of enlarged glands, accompanied by a great improvement in skin appearance, texture and smoothness. Sebaceous glands of the face and neck involute and regress in about two months. Also, sebaceous benign growths (circumscribed hyperplasia) of the forehead disappear.

### (c) Elimination of Demodex

Demodex folliculorum is a mite which strongly colonizes the facial follicles of older persons. The clinical signs are often subtle and go unrecognized by most physicians, including dermatologists. Usually, there is little more than scaling and follicular thickening, some perifollicular erythema, and small papules, associated with sensitive skin. Histology always shows perifollicular inflammation and sebaceous follicles crowded with mites. Invariably, application of soaps, perfumes, astringents, alcohols, mild acids (e.g., lactic acid) includes intense stinging, owing to the sub-clinical inflammatory reaction. Demodex infestation is very common, rarely diagnosed, but an important source of discomfort, contributing to a coarser appearance. It may be associated with small papulo-pustules. In short, when Demodex are numerous, typically so in sundamaged skin, there is subclinical disease.

Demodex takes its nourishment from sebum. It disappears after cis-retinoic acid treatment because of deprivation of lipoid nutrients. Topical retinoic acid, because it does not cause atrophy of sebaceous glands, has no effect on Demodex infestation.

### (d) Elimination of resident bacteria and yeasts

Propionibacteria are lipid dependent organisms which disappear when sebum secretion is reduced. They make a number of enzymes and products which are potentially harmful to skin, viz, lipases, proteases and comedogenic substances which produce horny impactions within the follicles (pseudo-comedones). All this contributes to poor skin texture and tolerance to irritants.

Pityrosporon species are follicular yeasts which induce folliculitis and a weakening of the follicular wall. They are also comedogenic, and add to follicular hyperkeratosis. They too are lipophilic organisms which disappear when the supply of sebum is reduced.

Oral cis-retinoic acid therapy results in follicular debridement with extrusion of retained horn. This elimination of horny material and vellus hairs also improves appearance since the follicular orifices become smaller and are not distended with retained products. A result of eliminating follicular contents, along with sebaceous gland shrinkage, is that the population of P. acnes, P. ovales and Demodex strikingly diminishes. Sensitivity to external stimuli goes down and the skin returns to a more normal state. Before treatment, swabbing the cheek with 10% lactic acid produces great stinging in many individuals. After treatment, stinging is absent or slight.

### (e) Actinic rhinophyma

Classical rhinophyma is an uneven enlargement of the nose due to increased deposition of collagen and enlargement of the sebaceous glands, usually an end-stage of chronic rosacea. The latter can reach huge size with a knobby appearance of the nose. Cis-retinoic acid, given orally, greatly reduces the volume of nasal tissue in this condition. We have also recognized a variant not associated with rosacea which we have called actinic rhinophyma. The nose becomes chronically reddened, with large, depressed pores, variably enlarged, uneven surface, accompanied by oiliness. It is quite common in males with photodamaged skin. Low-dosage oral cis-retinoic acid reduces the nasal enlargement with a marked improvement of the surface topography. Classic rhinophyma also responds to a substantial degree.

### (f) Hyperkeratosis and abnormal epithelial growths

The stratum corneum becomes focally thickened in aged skin, accentuating dryness and scaliness. The surface becomes rough, brittle and scaly. Oral cis-retinoic acid according to the invention thins down the horny layer and eliminates keratotic scale. The surface, therefore, becomes very much smoother. Incipient seborrheic keratoses (benign tumors) become less evident.

### (g) Epidermal atrophy

The viable epidermis becomes thin in the elderly; the cells are smaller and somewhat disorderly. Low dosage cis-retinoic acid induces thickening of the epidermis with larger, more normal-looking epidermal cells. A more normal, thicker epidermis contributes to a better textured skin with more fullness. These particular effects are like those of topical retinoic acid, but slower and of lesser magnitude. There occurs acanthosis (epidermal thickening), return to normal patterns, correction of dysplasia, atypia, etc. This results in smoother, nicer skin. Inhibition of tumor promotion is probable but has not been proved. We have observed that "flares" of subclinical actinic keratoses (pre-neoplastic growths) can be resolved.

### (h) Follicular papules and pustules

These are not acne but acneiform lesions. These are scattered pimples which might be a result of Demodex, bacteria (see above) or from cosmetics, soaps, etc. We are not referring to solar comedones either, though these too are reduced. These are inflammatory follicular lesions of varied origin. With oral cis-retinoic acid treatment according to the invention, they disappear in 2-3 months to the great satisfaction of the users.

### (i) Seborrheic-like dermatitis

This dermatitis is very common in older persons, especially males. This is an inflammatory condition which clears with low-dose oral cis-retinoic acid. Scaliness and redness regress.

As is known in the art, oral cis-retinoic acid therapy results in profound suppression of sebum production as a result of atrophy of the sebaceous glands, an effect not attainable by topical retinoic acid. Measurements of sebum production after 6 months of treatment show 45 to 65% reduction in all patients. Shrinkage of the sebaceous glands is reversible after stopping use of the drug. Moreover, oral cis-retinoic acid has an anti-inflammatory effect, moderating chronic dermatitis.

### (j) Facial lines and wrinkles

The dermatalogic literature does not recognize that finer alterations in facial texture, complexion, scaliness, etc. are amenable to cis-retinoic acid orally, at any dose. In fact, prior art treatments of various skin disorders with high doses of oral cis-retinoic acid have resulted in a worsening of the appearance and aesthetic quality of the skin, owing to excessive scaling and dryness.

With the oral 13-cis-retinoic acid treatment according to the invention, effacement of fine wrinkles has unexpectedly been observed. While applicants do not wish to be bound by any particular theory, it is believed that this treatment may result in a laying down or depositing of glycocollagen or glycoproteins in the epidermis, and/or possibly a rehydration of the horny layer. This appears to be a different mechanism from the effacement of lines and wrinkles with topical retinoic acid treatments.

In summary, the low-dose oral cis-retinoic acid therapy according to the present invention results in the following improvements in the skin:
1) Improved texture of the skin;
2) Reduced mottling-more uniform pigmentation;
3) Elimination of retained skin products in follicles, viz. hairs and horny material;
4) Control of follicular pustules and papules;
5) Shrinkage of sebaceous glands with disappearance of "stippled neck," knobby skin and sebaceous gland hyperplasia;
6) Reduction of facial skin oiliness in persons with excessive oiliness (seborrhea) to a pleasant normal skin appearance;
7) Reduction of nasal deformity in actinic rhinophyma;
8) Inhibition of actinic keratoses;
9) Improved skin turgor and elasticity;
10) Elimination of signs of seborrheic-like dermatitis, a common skin condition; and
11) Reduction of microbial population of bacteria and fungi, along with Demodex folliculorum.

Aside from the positive benefits described above for oral cis-retinoic acid therapy, laboratory studies with the present invention show none of the abnormalities commonly associated with the higher doses (40 to 80 mg) used for treating acne. Specifically, the following values have remained normal in all subjects: triglycerides, cholesterol, liver enzymes, SGOT and SGPT. These are elevated in one-third of patients receiving 80 mg daily. This greatly improves safety and eliminates problems in patients with a familial or personal history of heart disease, obesity, hypercholesteremia, etc.

Adverse muco-cutaneous reactions are very common with 40 to 80 mg doses. These include dry eyes, mouth and nose, cheilitis, itchy, and red friable skin. All of these are absent so that close monitoring by clinical and laboratory tests is unnecessary.

Laboratory follow-up of these patients reveals no changes in blood or urinary chemistries. SMA 12's have remained within normal limits. No adverse effects have been encountered, despite careful surveillance for signs of hypervitaminosis A. No toxicity of any kind has been encountered, and it is believed that the doses of the invention are below a teratogenic dose as well.

Quantitative bacteriology shows a sharp reduction in anaerobic bacteria, principally P. acnes, along with virtual disappearances of yeast-like fungi (P. species) The latter fungi contribute to seborrheic-like dermatitis in older persons. Cyanocrylate surface biopsies have shown elimination of the mite Demodex folliculorum.

The present invention may be embodied in other specific forms without departing from the spirit or essential attributes thereof, and, accordingly, reference should be made to the appended claims, rather than the specification, as indicating the scope of invention.

## Claims

1. The use of 13-cis-retinoic acid for the production of a medicament in an oral dosage form comprising 1 to 5 mg 13-cis-retinoic acid for the administration to women over child-bearing age and males over 50 years once per day or once every second day for retarding and reversing various age-related and associated disorders of the skin of elderly humans, said disorders being a mixture of conditions selected from sebaceous gland hyperplasia, distended sebaceous follicles, actinic rhinophyma, hyperkeratosis, elderly seborrheic-like dermatitis, demodex infestation of follicles, rosacea-like redness and swelling, epidermal atrophy, fine lines and wrinkles due to photoaging, accelerated hair loss due to dermatitis or inflammation associated with androgenic alopecia, and Pityrosporon-induced folliculites.

2. The use of 13-cis-retinoic acid according to claim 1 wherein the therapeutically effective amount is less than 2.5 mg daily.

3. The use of 13-cis-retinoic acid according to claim 1 wherein the therapeutically effective amount is 1 to 2 mg daily or every other day.

4. The use of 13-cis-retinoic acid according to claim 1 wherein the therapeutically effective amount is reduced to a maintenance dose of 1 to 5 mg two or three times a week after achieving therapeutic benefit.

5. The use of 13-cis-retinoic acid according to claim 1 wherein the oral administration is in capsule form.

## Patentansprüche

1. Verwendung von 13-cis-Retinsäure für die Herstellung eines Medikamentes in oraler Dosierungsform enthaltend 1 bis 5 mg 13-cis-Retinsäure zur Verabreichung an Frauen jenseits des Gebäralters und Männer über 50 Jahren einmal täglich oder einmal jeden zweiten Tag zur Verzögerung und Rückbildung verschiedener altersbedingter und mit dem Alter verbundener Störungen der Haut bei älteren Menschen, wobei diese Störungen aus einer Kombination von Zuständen von Talgdrüsenhyperplasie, erweiterten Talgdrüsenfollikeln, aktinischem Rhinophym, Hyperkeratose, seborrhoisch-ähnlicher Dermatitis der älteren Menschen, Demodexbefall von Follikeln, Rosazea-ähnlicher Rötung und Schwellung, Epidermal-Atrophie, feine Linien und Fältchen infolge von Altern durch Lichteinwirkung, beschleunigtem Haarausfall infolge von Dermatitis oder Entündung verbunden mit androgenetischer Alopezie und durch Pityrosporon-Hefen hervorgerufener Follikulitis ausgewählt sind.

2. Verwendung von 13-cis-Retinsäure gemäß Anspruch 1, wobei die therepeutisch wirksame Menge weniger als 2,5 mg täglich beträgt.

3. Verwendung von 13-cis-Retinsäure gemäß Anspruch 1, wobei die therepeutisch wirksame Menge 1 bis 2 mg täglich oder jeden zweiten Tag beträgt.

4. Verwendung von 13-cis-Retinsäure gemäß Anspruch 1, wobei die therapeutisch wirksame Menge auf eine Erhaltungsdosis von 1 bis 5 mg zwei- oder dreimal pro Woche nach Erlangung eines therapeutischen Erfolges reduziert ist.

5. Verwendung von 13-cis-Retinsäure gemäß Anspruch 1, wobei die orale Verabreichung in Kapselform erfolgt.

## Revendications

1. Emploi de l'acide 13-cis-rétinoïque pour la fabrication d'un médicament destiné à être administré par doses orales contenant de 1 à 5 mg d'acide 13-cis-rétinoïque, à des sujets féminins ayant dépassé l'âge de la maternité et à des sujets masculins de plus de cinquante ans, à raison d'une fois par jour ou d'une fois tous les deux jours, afin de retarder et faire régresser divers désordres de la peau dus à l'âge et qui se manifestent chez les sujets âgés, ces désordres correspondant à un mélange sélectif des conditions ci-après: hyperplasie des glandes sébacées, distension des follicules sébacés, rhinophyma actinique, hyperkératose, dermatite quasi séborrhéïque due à l'âge, infestation des follicules par des demodex, rougeur rosacée et inflammation associée, atrophie de l'épiderme, lignes fines et rides dues au vieillissement sous l'effet de la lumière, perte accélérée des cheveux due à une dermatite ou une inflammation associée à une alopécie androgénique, et folliculites induites par des pityrospores.

2. Emploi de l'acide 13-cis-rétinoïque selon la revendication 1, caractérisé en ce que la dose thérapeutiquement efficace est inférieure à 2,5 mg par jour.

3. Emploi de l'acide 13-cis-rétinoïque selon la revendication 1, caractérisé en ce que la dose thérapeutiquement efficace est comprise entre 1 et 2 mg par jour ou tous les deux jours.

4. Emploi de l'acide 13-cis-rétinoïque selon la revendication 1, caractérisé en ce qu'on réduit la dose thérapeutiquement efficace à une dose d'entretien de 1 à 5 mg, à raison de deux ou trois fois par semaine après avoir obtenu le bienfait thérapeutique.

5. Emploi de l'acide 13-cis-rétinoïque selon la revendication 1, caractérisé en ce que l'administration orale s'effectue sous forme de capsules.
